(19) Europäisches Patentamt
European Patent Office
Office européen des brevets

(11) Publication number: **0 073 837**
**B1**

(12) # EUROPEAN PATENT SPECIFICATION

(45) Date of publication of patent specification: **05.11.86**

(51) Int. Cl.[4]: **C 07 C 39/19,** C 07 C 37/18, C 07 C 43/23, C 07 C 47/56, C 07 C 47/565, C 07 C 45/00, C 07 C 45/43, C 07 C 49/647, C 07 C 49/683, C 07 C 49/687

(21) Application number: **82900665.9**

(22) Date of filing: **09.03.82**

(86) International application number: **PCT/JP82/00066**

(87) International publication number: **WO 82/03073 16.09.82 Gazette 82/22**

(54) **PROCESS FOR SELECTIVELY PRODUCING PARA-SUBSTITUTED DERIVATIVES OF PHENOLS.**

(30) Priority: **09.03.81 JP 32542/81**
**11.08.81 JP 125558/81**
**13.08.81 JP 127077/81**
**25.09.81 JP 151571/81**
**26.09.81 JP 152524/81**

(43) Date of publication of application: **16.03.83 Bulletin 83/11**

(45) Publication of the grant of the patent: **05.11.86 Bulletin 86/45**

(84) Designated Contracting States: **CH DE FR GB LI**

(56) References cited:
**FR-A-1 481 888**
**GB-A-1 197 168**
**JP-A-53 046 934**
**JP-B-26 003 729**
**JP-B-48 003 829**
**JP-B-53 135 943**

**The file contains technical information submitted after the application was filed and *not included in this specification***

(73) Proprietor: **Asahi Kasei Kogyo Kabushiki Kaisha**
**2-6, Dojimahama 1-chome Kita-ku**
**Osaka-shi Osaka 530 (JP)**

(72) Inventor: **HIRAI, Hidefumi**
**14-10, Yutenji 1-chome Meguro-ku**
**Tokyo 153 (JP)**
Inventor: **KOMIYAMA, Makoto**
**16-1-508, Kosuge 4-chome Katsushika-ku**
**Tokyo 124 (JP)**

(74) *Representative:* ***Schübel-Hopf, Ursula et al***
**Strehl, Schübel-Hopf, Schulz Patentanwälte**
**Widenmayerstrasse 17**
**D-8000 München 22 (DE)**

(56) References cited:
**Pharmazie, Vol. 33, Vol. 33, No. 7 (1978) Ohara M. et al. "Selective Reimer-Tiemann formylation of phenol via cychodextrin inclusion complex" P. 467**
**Die Makromolekulare Chemie Rapid *Communications*, Vol. 2, No. 2, (1981) (New York) Komiyama M. et al. "Selective syntheses of 2,5-cyclohexadienones catalyzed by beta-cyclodestrin" P. 177-178**

**EP 0 073 837 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Die Makromolekulare Chemie Rapid Communications, Vol. 2, No. 11, (1981) (New York) Komiyama M. et al. "Selective synthesis of 4-hydroxybenzoic acid with beta-cyclodextrin as catalyst" P. 661-662

Die Makromolekulare Chemie Rapid Communications, Vol. 2, No. 12, (1981) (New York) Komiyama. M. et al. "Selective synthesis of 4-hydroxybenyaldehyde with cyclodextrin as catalyst" P. 715-717

CHEMICAL ABSTRACTS, vol.96, no.4, February 1982, page 580, abstract no. 51854w, COLUMBUS, OHIO, (US)

CHEMICAL ABSTRACTS, vol. 96, no.4, February 1982, page 584, abstract no.51916t, COLUMBUS, OHIO, (US)

## Description

This invention relates to a process for selectively producing para-substituted derivatives of phenols. More particularly, the present invention is concerned with a process for producing para-substituted derivatives of phenols which comprises reacting a phenol compound with a haloform in the presence of an alkali metal hydroxide, using a cyclodextrin as a catalyst, while maintaining the molar ratio of the cyclodextrin to the haloform at 0.5 to 10, thereby to obtain the desired para-substituted derivative with an extremely high selectivity.

It is known that 2,5-cyclohexadienone derivatives having a dihalomethyl group at the 4-position or para-hydroxybenzaldehyde derivatives are prepared by reacting a phenol with a haloform under alkaline conditions. The products thus obtained are extremely valuable compounds as pharmaceuticals, agricultural chemicals, or raw materials for various physiologically active substances such as agricultural chemicals and pharmaceuticals, and dyes.

However, known reaction processes have serious disadvantages of extremely low selectivity and therefore poor yield. Accordingly, the known process cannot be advantageously used in practice.

For example, para-hydroxybenzaldehyde which, nowadays is of increasing importance as an anticarcinogen or a raw material for pharmaceuticals, agricultural chemicals and dyes, has conventionally been synthesized by reacting phenol with chloroform in the presence of an alkali. In the reaction, however, para-hydroxybenzaldehyde is obtained in a selectivity as low as about 30%, and a large amount of salicylaldehyde is formed as a by-product. Therefore, the production of para-hydroxybenzaldehyde in accordance with this process requires not only a large amount of raw materials but also an operation for separation.

In Pharmazie, Vol. 33, 467 (1978), it was reported that by the addition of β-cyclodextrin to the above-mentioned reaction system the selectivity for formation of para-hydroxybenzaldehyde is improved. However, in such a reaction since all the amount of chloroform is added at the time of initiation of the reaction in the customary manner, it is required to add the β-cyclodextrin in an amount of 0.75 in terms of molar ratio with respect to the phenol in order to attain a conversion of 19% and selectivity of 95%.

2,4-Dihydroxybenzaldehyde, nowadays, is also of increasing importance in view of its interesting behaviors such as cancer-controlling effect, plant root growth-promoting effect, antibacterial effect and photophosphorylation-controlling effect in chloroplast. For the production of 2,4-dihydroxybenzaldehyde, known is a process in which 1,3-dihydroybenzene is reacted with chloroform in the presence of an alkali. However, in this known process, a large amount of 2,4-dihydroxy-3-formylbenzaldehyde is formed as a by-product, and 2,4-dihydroxybenzaldehyde which is the intended product is obtained only in low yield and with low selectivity. Accordingly, for producing 2,4-dihydroxybenzaldehyde by this process, not only large amounts of raw materials but also an operation for separation is required.

2,5-Cyclohexadienone derivatives are highly reactive due to the conjugation of two C—C double bonds with a carbonyl group, and therefore are valuable as raw materials for the syntheses of physiologically active substances and other useful substances. Moreover, many of 2,5-cyclohexadienone derivatives themselves have physiological activities. Hitherto is known the Reimer-Tiemann reaction in which a para-substituted phenol is reacted with a haloform and sodium hydroxide or potassium hydroxide to give a 4-dihalomethyl-2,5-cyclohexadienone derivative. The reaction, however, gives compounds having substituents introduced mainly to the ortho-position. Therefore, the conventional process gives 2,5-cyclohexadienone derivatives in a yield as low as 5 to 10%, and requires not only large amounts of raw materials, but also a complicated operation for separation.

As described above, any of the conventional processes for introducing a substituent derived from a haloform to the para-position of phenols are unsatisfactory from a practical point of view because of low selectivity.

The present inventors have made extensive and intensive studies to develop a process in which a substituent derived from a haloform is introduced to the 4-position of phenols with high selectivity to give intermediates for the production of various useful substances as mentioned above. As a result, it has been found that, when a phenol compound is reacted with a haloform in the presence of a cyclodextrin under an alkaline condition while maintaining the molar ratio of the cyclodextrin to the haloform at 0.5 to 10, a substituent group derived from the haloform is introduced to the 4-position of the phenol compound with high selectivity, and therefore the intended para-substituted phenol derivative can be obtained in high yield. Based on such a novel finding, the present invention has been made.

According to the present invention, there is provided a process for producing a para-substituted phenol derivative which comprises reacting a phenol compound represented by the formula (I)

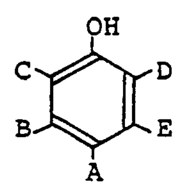

(I)

wherein A, B, C, D and E each independently stand for hydrogen, a hydroxyl group, a substituted or unsubstituted alkyl group, a substituted or unsubstituted allyl group, a substituted or unsubstituted alkoxyl group or a substituted or unsubstituted aryl group, provided that A does

not stand for a hydroxyl group and that when two or more of A, B, C, D and E each independently stand for a substituted or unsubstituted alkyl group or a substituted or unsubstituted alkoxyl group, they have their respective free terminal ends or at least one of them is bonded to another group selected from said alkyl and alkoxyl groups to form a ring, with a haloform in the presence of an alkali metal hydroxide, using as a catalyst a modified or unmodified cyclodextrin, thereby to selectively introduce an aldehyde group or dihalomethyl group derived from said haloform to the para-position of the phenol compound, characterized in that the reaction is effected while maintaining the molar ratio of the modified or unmodified cyclodextrin to the haloform at 0.5 to 10.

By the term "phenol compound" as used herein is meant phenol(hydroxybenzene) or its derivatives which is defined by the above-mentioned formula (I). The substituted or unsubstituted alkyl group, the substituted or unsubstituted allyl group, the substituted or unsubstituted alkoxyl gorup and the substituted or unsubstituted aryl group each may preferably have carbon atoms of not more than 6 with respect to the substituents B, C, D and E, and each may preferably have carbon atoms of not more than 12 with respect to the substituent A.

As the substituent to be introduced to the alkyl group, allyl group, alkoxyl group and aryl group, there can be mentioned an alkyl group, a halogen atom and others without specific restriction. However, too large a group is undesired.

The haloform may be used in an equimolar amount to the amount of phenol compound, but usually in an amount which is twice to 20 times the amount of the phenol compound, because the organic halide tends to decompose in an aqueous medium used as a reaction solvent, about which aqueous medium a description will be given later.

The alkali metal hydroxide to be used in the process of the present invention may preferably be sodium hydroxide or potassium hydroxide. The alkali metal hydroxide may be used in a stoichiometrical amount relative to the phenol compound. Usually, however, 1 to 15 times the stoichiometrical amount of the alkali metal hydroxide may be used taking into consideration of the rate of reaction and the like.

The reaction according to the process of the present invention is usually carried out in a reaction medium. As the reaction medium, there is employed an aqueous solvent, preferably water, because of the requirement that the reaction medium be capable of dissolving the alkali metal hydroxide therein. There may also be used, as the reaction medium, a mixture of water with a small amount of an organic solvent which is soluble in water and can be present stably under the reaction conditions. Examples of such an organic solvent include methanol, ethanol, acetone, dimethoxyethane and the like. The concentration of the alkali metal hydroxide in the reaction solvent may be in the range of 5 to 20% by weight, preferably 10 to 15% by weight.

A modified or unmodified cyclodextrin is used as a catalyst in the process of the present invention. Any of modified or unmodified α-, β and γ-cyclodextrins may be used. Usually, satisfactory results can be obtained by the use of unmodified α-, β- or γ-cyclodextrin. However, a more improved yield and selectivity in the reaction are achieved by the use of a modified α-, β- or γ-cyclodextrin of which the primary hydroxyl groups, for example, are all or partly substituted with a group which is stable under alkaline conditions, such as a N-methylformamido group. The abovementioned modified cyclodextrin may be prepared according to the method described in J. Amer. Chem. Soc., *102,* 762 (1980).

It is essential to the process of the present invention that the reaction of a phenol compound and a haloform be effected while maintaining the molar ratio of the cyclodextrin to the haloform in the reaction system at 0.5 to 10. The process of the present invention can be practiced by gradually adding a haloform to a system comprising a phenol compound, a cyclodextrin and an alkali metal hydroxide.

The reaction temperature is not critical, and the reaction may be carried out at room temperature or higher temperature, preferably at a temperature of from 50°C to 80°C.

The reaction time is also not critical, and may be suitably determined according to a phenol compound to be used, the amounts of reactants, reaction temperature, manner of addition of reactants and the like.

The reaction pressure is also not restricted, and the reaction is usually carried out at atmospheric pressure from a viewpoint of ease in operation.

By the reaction of a phenol compound with a haloform according to the process of the present invention, there is produced a para-substituted phenol derivative of the kind varied depending on the kinds of the phenol compound as described later.

From phenol compounds of the formula (I) in which A is a hydrogen atom, para-hydroxybenzaldehydes are obtained.

From phenol compounds of the formula (I) in which A is other substituent than hydrogen, namely, a substituted or unsubstituted alkyl group, a substituted or unsubstituted allyl group, a substituted or unsubstituted alkoxyl group or a substituted or unsubstituted aryl group, there are obtained 4-dihalomethyl-2,5-cyclohexadienone derivatives. Illustratively stated, when A in the formula (I) is hydrogen, there is obtained a para-substituted phenol derivative represented by the formula (II)

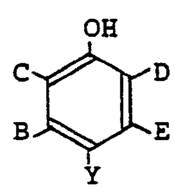

(II)

wherein B, C, D and E are as defined above and Y

stands for an aldehyde group, whereas when A in the formula (I) is a substituted or unsubstituted alkyl group, a substituted or unsubstituted allyl group, a substituted or unsubstituted alkoxyl group or a substituted or unsubstituted aryl group, there is obtained a para-substituted phenol derivative represented by the formula (III)

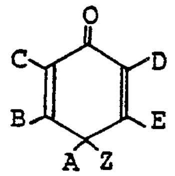

(III)

wherein A, B, C, D and E are as defined above, provided that A does not stand for hydrogen, and Z stands for haloform residue.

As is apparent from the foregoing, according to the process of the present invention, a variety of useful phenol derivatives having a substituent introduced to the para-position thereof are produced from phenols with high selectivity. Therefore, not only can be reduced the required amounts of phenols as raw materials but also the purification process can be extremely simplified, thus enabling the production process of the desired products to be economic.

The present invention will be illustrated in more detail with reference to the following Examples. Unless otherwise specified, reactions were carried out at atmospheric pressure in Examples and Comparative Examples as will be described hereinafter.

Example 1

In 50 ml of an aqueous 10% sodium hydroxide solution were dissolved 1.0 g (10.6 mmol) of phenol (first class grade reagent, manufactured and sold by Koso Chemical Co., Ltd., Japan) and 2.2 g (2.1 mmol) of α-cyclodextrin (special grade reagent, manufactured and sold by Nakarai Chemical Ltd., Japan). The resulting solution was heated to 60°C while agitating by means of a magnetic stirrer. Then, while dropwise adding 3 ml (37.4 mmol) of chloroform (special grade reagent, manufactured and sold by Tokyo Kasei, Co., Ltd. Japan) to the resulting solution, the reaction was allowed to proceed for 10 hours. Every 2 hours during the course of the reaction, part of the reaction mixture was taken, and subjected to the determination of chloroform contained therein by means of 701-type Gas Chromatograph manufactured by Ohkura Rikagaku Kenkyusho Co., Ltd., Japan (packing material, Porapak Q manufactured and sold by Gasukuro Kogyo Inc., Japan; column length, 2 m; column temperature, 30°C; carrier gas, helium). The analysis showed that the molar ratio of α-cyclodextrin to chloroform was 0.9 to 1.6. After completion of the reaction, the reaction mixture was acidified with hydrochloric acid, and subjected to extractions each with 50 ml of ethyl ether 5 times. The ethyl ether layer was washed with water, and then evaporated to dryness, thereby to obtain 1.1 g of a solid. The solid thus obtained was treated with benzene to give 0.6 g of a benzene-insoluble matter and 0.5 g of a benzene-soluble matter. The infrared spectrum of the benzene-insoluble matter was in agreement with that of para-hydroxybenzaldehyde (special grade reagent, manufactured by Tokyo Kasei Co., Ltd., Japan). On the other hand, the spectrum of the benzene-soluble matter was in agreement with that of phenol. Salicylaldehyde was observed neither in the benzene-soluble matter nor in the benzene-insoluble matter. The yield of the intended product was 46% and the selectivity was 92%.

In this example, β-cyclodextrin was used in an amount of 0.2 in terms of molar ratio with respect to the phenol.

Comparative Example 1

In 50 ml of an aqueous 10% sodium hydroxide solution was dissolved 1.0 g (10.6 mmol) of phenol (first class grade reagent, manufactured and sold by Koso Chemical Co., Ltd., Japan). The resulting solution was heated to 60°C while agitating by means of a magnetic stirrer. Then, while dropwise adding 3 ml (37.4 mmol) of chloroform (special grade reagent, manufactured and sold by Tokyo Kasei Co., Ltd., Japan) to the resulting solution, the reaction was allowed to proceed for 1.0 hours. After completion of the reaction, the reaction mixture was acidified with hydrochloric acid, and subjected to extractions each with 50 ml of ethyl ether 5 times. The ethyl ether layer was washed with water, and then evaporated, thereby to obtain 1.0 g of an oily material. The oily material thus obtained was analyzed by means of 701-type Gas Chromatograph manufactured by Ohkura Rikagaku Kenkyusho Co., Ltd., Japan (packing material, Uniport HP manufactured and sold by Gasukuro Kogyo Inc., Japan; column length, 2 m; column temperature, 140°C; carrier gas, helium). The analysis showed that the oily material was a mixture of 0.2 g (1.6 mmol) of para-hydroxybenzaldehyde, 0.4 g (3.2 mmol) of salicylaldehyde and 0.4 g of phenol. Namely, the yield of the intended product was 15% and the selectivity was 25%.

In this example, cyclodextrin was not used.

Comparative Example 2

In 50 ml of an aqueous 10% sodium hydroxide solution were dissolved 1.0 g (10.6 mmol) of phenol (first class grade reagent, manufactured and sold by Koso Chemical Co., Ltd., Japan) and 2.2 g (2.1 mmol) of α-cyclodextrin (special grade reagent, manufactured and sold by Nakarai Chemical Ltd., Japan). To the resulting solution was added 3 ml (37.4 mmol) of chloroform (special grade reagent, manufactured and sold by Tokyo Kasei Co., Ltd., Japan). Namely, the molar ratio of α-cyclodextrin to chloroform was 0.06 at the start of the reaction. The solution was heated at 60°C for 10 hours while agitating by means of a magnetic stirrer. After completion of the reaction, the reaction mixture was acidified with hydrochloric acid, and subjected to extractions each with 50 ml of ethyl ether 5 times. The ethyl ether layer was washed with water, and then evaporated, thereby to obtain 1.1 g of an oily material. The oily material thus obtained was analyzed by means of 701-type Gas Chromatograph manufactured by Ohkura Rikagaku Kenkyusho Co., Ltd., Japan (packing material, Uniport HP manufactured and sold by Gasukuro Kogyo Inc., Japan; column length, 2 m; column temperature, 140°C; carrier gas, helium). The analysis showed that the oily material was a mixture of 0.4 g (3.2 mmol) of para-hydroxybenzaldehyde, 0.3 g (2.4 mmol) of salicylaldehyde and 0.4 g of phenol. Namely, the yield of the intended product was 30% and the selectivity was 51%.

In this example α-cyclodextrin was used in an amount of 0.2 in terms of molar ratio with respect to the phenol, which was the same as in Example 1.

Example 2

Substantially the same procedures as in Example 1 were repeated except that 2.4 g (2.1 mmol) of β-cyclodextrin (special grade reagent, manufactured and sold by Nakarai Chemical Ltd., Japan) was used instead of 2.2 g of α-cyclodextrin. There was obtained 0.5 g (4.0 mmol) of para-hydroxybenzaldehyde, with 0.6 g of unreacted phenol recovered. Salicylaldehyde was not detected. Namely, the yield of the intended product was 39% and the selectivity was 98%.

In this example, β-cyclodextrin was used in an amount of 0.2 in terms of molar ratio with respect to the phenol.

Comparative Example 3

Substantially the same procedures as in Comparative Example 2 were repeated except that 2.4 g (2.1 mmol) of β-cyclodextrin (special grade reagent, manufactured and sold by Nakarai Chemical Ltd., Japan) was used instead of 2.2 g of α-cyclodextrin. Namely, the molar ratio of β-cyclodextrin top chloroform was 0.06 at the start of the reaction. There was obtained a mixture of 0.3 g (2.4 mmol) of para-hydroxybenzaldehyde, 0.2 g (1.6 mmol) of salicylaldehyde and 0.5 g of phenol. Namely, the yield of the intended product was 23% and the selectivity was 45%.

In this example, β-cyclodextrin was used in an amount of 0.2 in terms of molar ratio with respect to the phenol, which was the same as in Example 2.

Example 3

In 60 ml of an aqueous 10% sodium hydroxide solution were dissolved 1.0 g (9.1 mmol) of 1,3-dihydroxybenzene (special grade reagent, manufactured and sold by Tokyo Kasei Co., Ltd., Japan) and 8.0 g (7.0 mmol) and β-cyclodextrin (special grade reagent, manufactured and sold by Nakarai Chemical Ltd., Japan). While gradually, dropwise adding 5 ml (62.3 mmol) of chloroform (special grade reagent, manufactured and sold by Tokyo Kasei Co., Ltd., Japan) to the resulting solution and agitating by means of a magnetic stirrer, the reaction was allowed to proceed at 60°C for 4 hours. After completion of the reaction, the reaction mixture was acidified with hydrochloric acid and subjected by ex tractions each with 50 ml of ethyl ether 3 times. The ethyl ether layer was washed with water, and then dried, thereby to obtain 1.2 g of a product. The product thus obtained was analyzed by means of 701-type Gas Chromatograph manufactured by Ohkura Rikagaku Kenkyusho Co., Ltd., Japan (packing material, Tenax GC manufactured and sold by Gasukuro Kogyo Inc., Japan; column length, 2 m; column temperature, 300°C; carrier gas, helium). The analysis showed that the product was entirely 2,4-dihydroxybenzaldehyde, without any 2,4-dihydroxy-3-formylbenzaldehyde detected. Namely, the yield of the intended product was 96% and the selectivity was 100%.

Comparative Example 4

Each reagent used herein was of the same grade as in Example 2. In 60 ml of an aqueous 10% sodium hydroxide solution was dissolved 1 g (9.1 mmol) of 1,3-dihydroxybenzene. While gradually, dropwise adding 5 ml (62.3 mmol) of chloroform to the resulting solution and agitating by means of a magnetic stirrer, the reaction was allowed to proceed at 60°C for 4 hours. After completion of the reaction, the reaction mixture was acidified with hydrochloric acid, and subjected to extractions each with 50 ml of ethyl ether 3 times. The ethyl ether layer was washed with water, and then dried, thereby to obtain 0.9 g of a product. The product thus obtained was analyzed by means of 701-type Gas Chromatograph manufactured by Ohkura Rikagaku Kenkyusho Co., Ltd., Japan. The analysis showed that the product was a mixture of 0.3 g of 2,4-dihydroxybenzaldehyde and 0.6 g of 2,4-dihydroxy-3-formylbenzaldehyde. Namely, the yield of the intended product was 24% and the selectivity was 38%.

Example 4

In 50 ml of an aqueous 10% sodium hydroxide solution were dissolved 1 g of p-cresol and 8 g of β-cyclodextrin. To the resulting solution, 5 ml of chloroform was gradually, dropwise added at 75°C, and allowed to react for 10 hours. After completion of the reaction, the reaction mixture was subjected to extractions each with 50 ml of chloroform 5 times, and then the chloroform layer was dried, thereby to obtain 0.75 g of a product. The $^1H$—NMR measurement showed that the product thus obtained was entirely 4-dichloromethyl-4-methyl-2,5-cyclohexadienone. Namely, the yield of the intended product was 43% and the selectivity was 100%.

Comparative Example 5

In 50 ml of an aqueous 10% sodium hydroxide solution was dissolved 1 g of p-cresol. To the resulting solution, 5 ml of chloroform was gradually, dropwise added to 75°C and allowed to

react for 10 hours. After completion of the reaction, the reaction mixture was subjected to extractions each with 50 ml of chloroform 5 times. The chloroform layer was dried to obtain 0.29 g of product. The product thus obtained was a mixture of 28% of 4-dichloromethyl-4-methyl-2,5-cyclohexadienone, and 72% of 2-formyl-4-methylphenol. Namely, the yield of the intended product was 5% and the selectivity was 28%.

Example 5

Substantially the same procedures as in Example 4 were repeated except that 1 g of 4-phenylphenol was used instead of 1 g of p-cresol. There was obtained 0.89 g of a product. The $^1H$—NMR measurement showed that 95% of the product was 4-dichloromethyl-4-phenyl-2,5-cyclohexadienone and the balance was 2-formyl-4-phenylphenol. Namely, the yield of the intended product was 60% and the selectivity was 95%.

Comparative Example 6

Substantially the same procedures as in comparative Example 5 were repeated except that 1 g of 4-phenylphenol was used instead of 1 g of p-cresol. There was obtained 0.50 g of a product. The product was a mixture of 12% of 4-dichloromethyl-4-phenyl-2,5-cyclohexadienone and 88% of 2-formyl-4-phenylphenol. Namely, the yield of the intended product was 4% and the selectivity was 12%.

Example 6

Substantially the same procedures as in Example 4 were repeated except that 1 g of 3,4,5-trimethylphenol was used instead of 1 g of p-cresol. There was obtained 0.90 g of a product. The product thus obtained was entirely 3,4,5-trimethyl-4-dichloromethyl-3,5-cyclohexadienone. Namely, the yield of the intended product was 56% and the selectivity was 100%.

Comparative Example 7

Substantially the same procedures as in Comparative Example 5 were repeated except that 1 g of 3,4,5-trimethylphenol was used instead of 1 g of p-cresol. There was obtained 0.38 g of a product. The product thus obtained was a mixture of 5% of 3,4,5-trimethyl-4-dichloromethyl-2,5-cyclohexadienone and 95% of 3,4,5-trimethyl-2-formylphenol. Namely, the yield of the intended product was 1% and the selectivity was 13%.

Example 7

Substantially the same procedures as in Example 4 were repeated except that 1 g of ar-2-tetrahydronaphthol was used instead of 1 g of p-cresol. There was obtained 0.56 g of 9-dichloromethyl-6-oxo-1,2,3,4,6,9-hexahydronaphthalene. Namely, the yield of the intended product was 36% and the selectivity was 100%.

Comparative Example 8

Substantially the same procedures as in Comparative Example 5 were repeated except that 1 g of ar-2-tetrahydronaphthol was used instead of 1 g of p-cresol. There was obtained 0.52 g of a product. The product thus obtained was a mixture of 19% of 9-dichloromethyl-6-oxo-1,2,3,4,6,9-hexahydronaphthalene and 81% of 1-formyl-ar-2-tetrahydronaphthol. Namely, the yield of the intended product was 6% and the selectivity was 19%.

Example 8

Substantially the same procedures as in Example 4 were repeated except that 1 g of 2,4,6-trimethylphenol was used instead of 1 g of p-cresol. There was obtained 0.75 g of 2,4,6-trimethyl-4-dichloromethyl-2,5-cyclohexadienone. Namely, the yield of the intended product was 47% and the selectivity was 100%.

Example 9

Substantially the same procedures as in Example 4 were repeated except that 1 g of p-methoxyphenol was used instead of 1 g of p-cresol. There was obtained 0.31 g of a product. The product thus obtained was a mixture of 96% of 4-dichloromethyl-4-methoxy-2,5-cyclohexadienone and 4% of 2-formyl-4-methoxyphenol. Namely, the yield of the intended product was 27% and the selectivity was 96%.

Example 10

Substantially the same procedures as in Example 7 were repeated except that 8 g of α-cyclodextrin was used instead of 8 g of β-cyclodextrin. From 1 g of ar-2-tetrahydronaphthol, there was obtained 0.41 g of a product. The product contained 9-dichloromethyl-6-oxo-1,2,3,4,6,9-hexahydronaphthalene in an amount of 93%. Namely, the yield of the intended product was 24% and the selectivity was 93%.

Example 11

Substantially the same procedures as in Example 4 were repeated except that an aqueous 10% potassium hydroxide solution was used instead of an aqueous 10% sodium hydroxide solution. From 1 g of p-cresol, there was obatined 0.52 g of 4-dichloromethyl-4-methyl-2,5-cyclohexadienone. Namely, the yield of the intended product was 29% and the selectivity was 100%.

**Claims**

1. A process for selectively producing para-substituted phenol derivatives of phenols which comprises reacting a phenol compound represented by the formula (I)

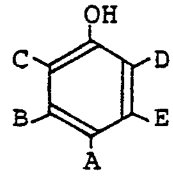

(I)

wherein A, B, C, D and E each independently

stand for hydrogen, a hydroxyl group, a substituted or unsubstituted alkyl group, a substituted or unsubstituted allyl group, a substituted or unsubstituted alkoxyl group or substituted or unsubstituted aryl group, provided that A does not stand for a hydroxyl group and that when two or more of A, B, C, D, and E each independently stand for a substituted or unsubstituted alkyl group or a substituted or unsubstituted alkoxyl group, they have their respective free terminal ends or at least one of them is bonded to another group selected from said alkyl and alkoxyl groups to form a ring, with a haloform in the presence of an alkali metal hydroxide, using a catalyst a modified or unmodified cyclodextrin, thereby to introduce an aldehyde group or a dihalomethyl group derived from said haloform to the para-position of the phenol compound, characterized in that the reaction is effected while maintaining the molar ratio of said modified or unmodified cyclodextrin to said haloform at 0.5 to 10.

2. A process according to claim 1, wherein said substituted or unsubstituted alkyl group, said substituted or unsubstituted allyl group, said substituted or unsubstituted alkoxyl group and said substituted or unsubstituted aryl group each have carbon atoms of not more than 6 with respect to B, C, D and E and each have carbon atoms of not more than 12 with respect to A.

3. A process according to claim 1, wherein said alkali metal hydroxide is sodium hydroxide or potassium hydroxide.

4. A process according to claim 1, wherein the reaction is effected by gradually adding the haloform to a system comprising the phenol compound, the alkali metal hydroxide and the modified or unmodified cyclodextrin.

5. A process according to claim 1, wherein the reaction is effected in an aqueous medium.

6. A process according to claim 1, wherein A in the formula (I) is hydrogen and said para-substituted phenol derivative is represented by the formula (II)

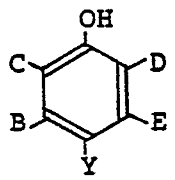

(II)

wherein B, C, D and E are as defined above and Y stands for an aldehyde group, or A in the formula (I) is substituted or unsubstituted alkyl group, a substituted or unsubstituted allyl group, a substituted or unsubstituted alkoxyl group or a substituted or unsubstituted aryl group and said para-substituted phenol derivative is represented by the formula (III)

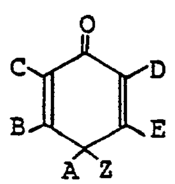

(III)

wherein A, B, C, D and E are as defined above, provided that A does not stand for hydrogen, and Z stands for a haloform residue.

**Patentansprüche**

1. Verfahren zur selektiven Herstellung von para-substituierten Derivaten von Phenolen, bei dem eine durch die Formel (I) dargestellte Phenolverbindung

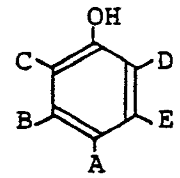

(I)

in der A, B, C, D und E jeweils unabhängig voneinander für Wasserstoff, eine Hydroxygruppe, eine substituierte oder unsubstituierte Alkylgruppe, eine substituierte oder unsubstituierte Allylgruppe, eine substituierte oder unsubstituierte Alkoxygruppe oder eine substituierte oder unsubstituierte Arylgruppe stehen, vorausgesetzt, daß A nicht für eine Hydroxylgruppe steht und daß dann, wenn zwei oder mehr der Symbole A, B, C, D und E jeweils unabhängig voneinander für eine substituierte oder unsubstituierte Alkylgruppe oder eine substituierte oder unsubstituierte Alkoxygruppe stehen, diese entsprechende freie terminale Enden haben oder mindestens eine unter ihnen an eine andere Gruppe, die unter diesen Alkyl- under Alkoxygruppen ausgewählt ist, unter Ringbildung gebunden ist, mit einem Haloform in Gegenwart eines Alkalimetallhydroxids unter Verwendung eines modifizierten oder unmodifizierten Cyclodextrins als Katalysator umgesetzt wird, wobei eine Aldehydgruppe oder eine Dihalomethylgruppe, die von dem Haloform abgeleitet ist, in die Para-Stellung der Phenolverbindung eingeführt wird, dadurch gekennzeichnet, daß die Reaktion durchgeführt wird, während das Molverhältnis des modifizierten oder unmodifizierten Cyclodextrins zu dem Haloform bei 0,5 bis 10 gehalten wird.

2. Verfahren nach Anspruch 1, bei dem die substituierte oder unsubstituierte Alkylgruppe, die substituierte oder unsubstituierte Allylgruppe, die substituierte oder unsubstituierte Alkoxygruppe und die substituierte oder unsubstituierte Arylgruppe jeweils nicht mehr als 6 Kohlenstoffatome aufweisen, whenn sie für B, C, D und E stehen und jeweils nicht mehr als 12 Kohlenstoffatome haben, wenn sie für A stehen.

3. Verfahren nach Anspruch 1, bei dem das Alkalimetallhydroxid Natriumhydroxid oder Kaliumhydroxid ist.

4. Verfahren nach Anspruch 1, bei dem die Reaktion durchgeführt wird, indem das Haloform allmählich zu einem System zugefügt wird, welches die Phenolverbindung, das Alkalimetallhydroxid und das modifizierte oder unmodifizierte Cyclodextrin enthält.

5. Verfahren nach Anspruch 1, bei dem die

Reaktion in einem wässrigen Medium durchgeführt wird.

6. Verfahren nach Anspruch 1, bei dem A in Formel (I) Wasserstoff ist und das para-substituierte Phenolderivate durch die Formel (II) dargestellt wird

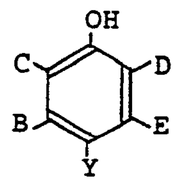

(II)

in der B, C, D und E die vorstehende Definition haben und Y für eine Aldehydgruppe steht, oder A in Formel (I) eine substituierte oder unsubstituierte Alkylgruppe, eine substituierte oder unsubstituierte Allylgruppe, eine substituierte oder unsubstituierte Alkoxygruppe oder eine substituierte oder unsubstituierte Arylgruppe ist und das para-substituierte Phenolderivat durch die Formel (III) dargestellt wird

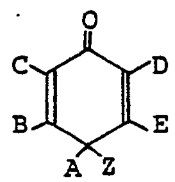

(III)

in der A, B, C, D und E die vorstehende Definition haben, vorausgesetzt, daß A nicht für Wasserstoff steht, und Z für einen Haloformrest steht.

**Revendications**

1. Un procédé pour produire sélectivement des dérivés parasubstitués de phénols, qui comprend une réaction d'un composé de phénol représenté par la formule (I)

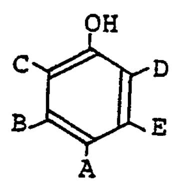

(I)

dans laquelle A, B, C, D et E représentent indépendamment l'hydrogène, un groupe hydroxyle, un groupe alcoyle substitué ou non substitué, un groupe allyle substitué ou non substitué, un groupe alcoxyle substitué ou non substitué et un groupe aryle substitué ou non substitué, à la condition que A ne représente pas un groupe hydroxyle et que quand deux ou un plus grand nombre des radicaux A, B, C, D et E représentent chacun indépendamment un groupe alcoyle substitué ou non substitué ou un groupe alcoxyle substitué ou non substitué, leurs extrémités terminales libres respectives, ou au moins l'une d'elles, sont liées, à un autre groupe choisi parmi lesdits groupes alcoyle et alcoxyle pour former un noyau, avec un halogènoforme en présence d'un hydroxyde de métal alcalin, avec utilisation, comme catalyseur, d'une cyclodextrine modifiée ou non modifiée, en introduisant ainsi un groupe aldéhyde ou un groupe dihalométhyle dérivé dudit halogénoforme en position para du composé du phénol caratérisé en ce que le réaction est effectuée tout en maintenant le rapport molaire de ladite cyclodextrine modifiée ou non modifiée audit halogènoforme dans une plage de 0,5 à 10.

2. Un procédé selon la revendication 1, dans lequel ledit groupe alcoyle substitué ou non substitué, ledit groupe allyle substitué ou non substitué, ledit groupe alcoxyle substitué ou non substitué et ledit groupe aryle substitué ou non substitué on chacun un nombre d'atomes de carbone non supérieur à 6 en ce qui concerne B, C, D, et E et ont chacun nombre d'atomes de carbone non supérieur à 12 en ce qui concerne A.

3. Un procédé selon la revendication 1, dans lequel ledit hydroxyde de métal alcalin est l'hydroxyde de sodium ou l'hydroxyde de potassium.

4. Un procédé selon la revendication 4, dans lequel la réaction est effectuée en ajoutant graduellement l'halogènoforme à un système comprenant le composé du phénol, l'hydroxyde de métal alcalin et la cyclodextrine modifiée ou non modifiée.

5. Un procédé selon la revendication 1, dans lequel la réaction est effectuée dans un milieu aqueux.

6. Un procédé selon la revendication 1, dans lequel A dans la formule (I) est l'hydrogène et ledit dérivé para-substitué du phénol est représenté par la formule (II)

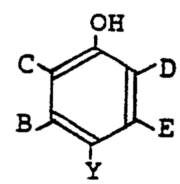

(II)

dans laquelle B, C, D et E ont les significations définies ci-dessus et Y représente un groupe aldéhyde, ou A dans la formule (I) est un groupe alcoyle substitué ou non substitué, un groupe allyle substitué ou non substitué, un groupe alcoxyle substitué ou non substitué ou un groupe aryle substitué ou non substitué et ledit dérivé para-substitué du phénol est représenté par la formule (III)

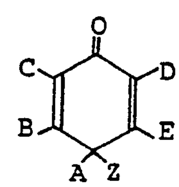

(III)

dans laquelle A, B, C, D et E ont les significations définies ci-dessus, à la condition que A ne représente pas l'hydrogène, et Z représente un résidu d'halogénoforme.